Europäisches Patentamt

European Patent Office

Office européen des brevets

(11) Numéro de publication: **0 192 551 B1**

(12) **FASCICULE DE BREVET EUROPEEN**

(45) Date de publication de fascicule du brevet: **19.11.92**  (51) Int. Cl.⁵: **G01N 25/04**, G01N 33/28

(21) Numéro de dépôt: **86400263.9**

(22) Date de dépôt: **07.02.86**

(54) **Procédé et appareil pour la détermination du point de décongélation des carburéacteurs.**

(30) Priorité: **12.02.85 FR 8501944**

(43) Date de publication de la demande:
**27.08.86 Bulletin 86/35**

(45) Mention de la délivrance du brevet:
**19.11.92 Bulletin 92/47**

(84) Etats contractants désignés:
**AT BE CH DE GB IT LI LU NL SE**

(56) Documents cités:
**EP-A- 0 041 458**
**GB-A- 1 438 754**

**PATENT ABSTRACTS OF JAPAN, vol. 6, no.**
**88 (P-118)[966], 26 mai 1982;& JP-A-57 24 850**

(73) Titulaire: **TOTAL RAFFINAGE DISTRIBUTION S.A.**
**84, rue de Villiers**
**F-92300 Levallois Perret(FR)**

(72) Inventeur: **Bock, Patrice**
**11, Rue Jules Siegfried**
**F-76600 Le Havre(FR)**
Inventeur: **Dutot, Philippe**

**F-76280 Fongueusemare(FR)**

(74) Mandataire: **Jolly, Jean-Pierre et al**
**Cabinet Jolly 54, rue de Clichy**
**F-75009 Paris(FR)**

## Description

Les carburéacteurs utilisés en aviation doivent présenter des caractéristiques de tenue au froid très précises, réglementées par des normes internationales. Ainsi, la température d'apparition des cristaux lors du refroidissement du carburéacteur et surtout la température de disparition des cristaux (point de décongélation) au réchauffage sont des spécifications importantes. La norme admet au plus un écart de 3°C entre des températures d'apparition et disparition des cristaux.

On connaît déjà un dispositif pour la détermination du point de décongélation, ce dispositif comprenant un vase Dewar rempli d'un liquide réfrigérant dans lequel est plongée une éprouvette étanche contenant une quantité prédéterminée de carburéacteur, un agitateur et un thermomètre. L'aspect du produit est contrôlé visuellement en fonction de la température. A l'apparition d'un trouble, à la descente en température, on conclue à l'apparition des cristaux, et on note cette température; la disparition de ceux-ci se traduit par un retour à la limpidité, et on note à nouveau cette température.

Cependant, le temps nécessaire à un opérateur pour déterminer le point de décongélation par ce procédé manuel est trop long. De plus, la répétabilité de ce procédé, est relativement élevée, puisqu'elle est de l'ordre de 0,7°C.

La présente invention a pour but de remédier aux inconvénients du procédé manuel ci-dessus décrit et concerne à cet effet un procédé pour la détermination automatique du point de décongélation d'un carburéacteur, qui se caractérise en ce qu'il consiste :

- à soumettre un échantillon du carburéacteur à un cycle thermique au cours duquel la température est diminuée jusqu'à la température d'apparition des cristaux, puis est relevée de façon que les cristaux disparaissent;
- à effectuer une analyse thermique dudit carburéacteur en mesurant à des intervalles de temps réguliers, au cours dudit cycle, la température que prend le carburéacteur;
- à effectuer simultanément une analyse optique du carburéacteur en mesurant aux mêmes moments l'intensité de lumière transmise à travers ledit échantillon ;
- à transformer lesdites mesures respectivement en signaux de détection thermique et en signaux de détection optique, et
- à envoyer lesdits signaux à une unité centrale de traitement des données, telle qu'un microprocesseur, qui calcule à partir de ces signaux les températures d'apparition de cristaux par détection thermique et par détection optique, et les températures de disparition de cristaux par ces deux types de détection, pour déduire à partir de ces valeurs le point de décongélation dudit carburéacteur.

L'apparition de cristaux modifie, par diffraction, l'état de transmission de la lumière à travers l'échantillon, et à la disparition des cristaux, on retrouve les caractéristiques optiques d'origine.

Le procédé selon l'invention met donc en jeu un double système de détection de la température de disparition des cristaux. Le contrôle de la cohérence des résultats à l'aide de l'unité de traitement de données assure une précision au moins équivalente, si ce n'est supérieure, à celle de la détection visuelle selon la technique antérieure.

Ce procédé à double détection est en outre plus fiable qu'un procédé à simple détection puis qu'une détermination purement thermique pourrait laisser inaperçue une légère pollution du carburéacteur, et qu'une détermination purement optique pourrait conclure à des non conformités du carburéacteur à la suite d'un simple dépôt de givre sur la paroi extérieure du récipient qui contient l'échantillon de carburéacteur.

Enfin, le temps d'occupation d'un opérateur pour la mise en oeuvre du procédé selon l'invention ne dépasse pas une dizaine de minutes, alors qu'il est d'environ 1 heure pour le procédé manuel.

Les mesures de la température et de l'état de transmission de la lumière peuvent être effectuées toutes les secondes et l'analyse de l'échantillon peut être poursuivie jusqu'à une température négative limite, par exemple jusqu'à -65°C établie par rapport à la température la plus basse que peut rencontrer un aéronef en cours de vol. Si aucun phénomène, apparition ou disparition de cristaux, n'est observé, l'unité de traitement émet un message correspondant.

L'unité de traitement vérifie également que les températures d'apparition de cristaux déterminées par les deux types de détection ne diffèrent pas entre elles de plus d'une valeur prédéterminée, prise par exemple égale à la répétabilité du procédé manuel. Une vérification analogue est effectuée pour les températures de disparition des cristaux. Si cette différence est trouvée supérieure à ladite valeur prédéterminée, l'unité de traitement émet un message indiquant que le carburéacteur est vraisemblablement pollué.

De plus, l'unité de traitement vérifie si la moyenne des températures de disparition de cristaux par la détection thermique et par la détection optique, et la moyenne des températures d'apparition de cristaux par ces deux types de détection ne diffèrent pas de plus d'une seconde valeur prédéterminée, fixée par les

2

normes, par exemple de 3°C. Si cette condition n'est pas vérifié, l'unité de traitement indique que la mesure n'est pas conforme à la norme, et émet un message suggérant de recommencer l'essai.

La présente invention concerne également un appareil pour la détermination du point de décongélation d'un carburéacteur par le procédé susmentionné, ledit dispositif étant caractérisé en ce qu'il comprend :

- une éprouvette en matière transparente contenant une quantité prédéterminée du carburéacteur, dans laquelle plongent un agitateur et une sonde de température ;
- une source de fluide caloporteur destiné à réchauffer l'éprouvette, dès que la température d'apparition de cristaux a été détectée par l'unité centrale;
- une enveloppe réfrigérante également en matière transparente, qui entoure ladite éprouvette et dans laquelle est entretenue au moyen d'une pompe, une circulation d'un fluide frigoporteur fournie par la source de froid ;
- un détecteur optique comprenant une source émettrice d'un faisceau de lumière conduit par une première fibre optique et un dispositif récepteur de l'intensité lumineuse transmise, conduite par une seconde fibre optique, ces deux fibres optiques étant disposées dans l'axe de l'éprouvette, la première à l'extérieur de l'éprouvette du côté du fond et la seconde à l'intérieur de celle-ci juste sous la surface du carburéacteur ;
- et une unité centrale de traitement de données, telle qu'un microprocesseur, destinée à calculer d'une part, les températures d'apparition de cristaux à partir des mesures fournies par la sonde de température et des mesures fournies par le détecteur optique, et d'autre part, les températures de disparition des cristaux à partir de ces deux types de mesure, et d'en déduire le point de décongélation dudit carburéacteur.

Une extrémité de la fibre optique réceptrice est située dans l'éprouvette sous la surface du carburéacteur, de préférence au foyer de la lentille constituée par le fond de l'éprouvette et par le carburéacteur.

Avantageusement, l'appareil selon l'invention comprend également une horloge permettant, à des intervalles de temps réguliers, l'acquisition de la température (au centième de degré Celsius près) et de l'état de transmission optique (par rapport à un seuil déterminé), un affichage numérique de la température instantanée mesurée et une imprimante pour l'édition des résultats.

La mise en circulation du fluide frigoporteur à travers l'enveloppe réfrigérante ainsi que l'injection de fluide caloporteur sont commandées par l'unité centrale de traitement de données.

L'invention sera à présent expliquée en détail en regard des dessins annexés dans lesquels :

La figure 1 représente une vue en coupe axiale d'une cellule de mesure de l'appareil selon l'invention ;

La figure 2 est une courbe typique d'analyse thermique d'un carburéacteur ;

Les figures 3 et 4 sont respectivement les représentations d'une fonction qui traduit les variations de la dérivée seconde, au voisinage du point d'apparition des cristaux et au voisinage du point de disparition des cristaux.

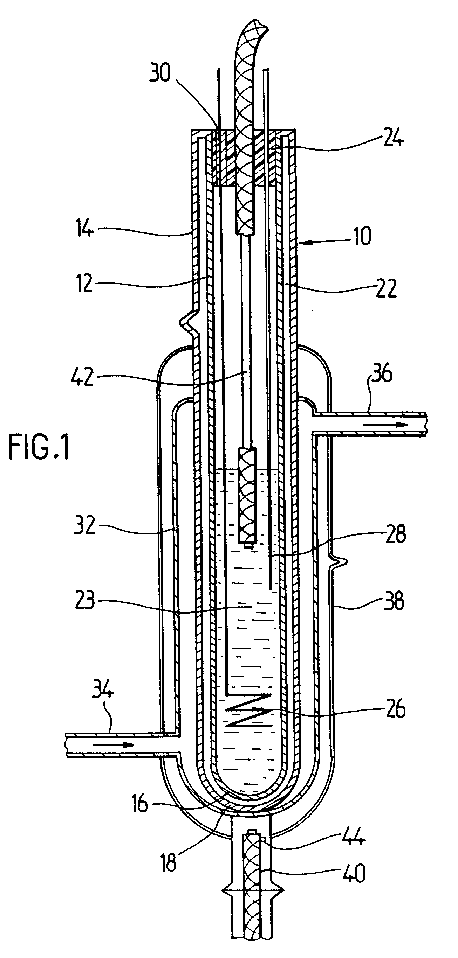

L'appareil pour la détermination automatique du point de décongélation d'un carburéacteur comprend essentiellement une cellule de mesure dont un exemple de réalisation est illustré par la figure 1.

Cette cellule comprend une éprouvette 10 à double enveloppe formée de deux tubes coaxiaux 12, 14 à fonds 16, 18 hémisphériques et réalisés en une matière transparente. L'espace 22 compris entre les deux tubes est rempli d'air sec ou d'azote sec.

L'éprouvette est remplie d'un volume prédéterminé de carburéacteur 23, se présentant sous forme liquide dans les conditions normales de température et de pression. L'éprouvette est disposée verticalement et est obturée hermétiquement à son ouverture supérieure par un bouchon 24.

Dans le carburéacteur plongent un agitateur 26 et une sonde de température 28. L'agitateur est entraîné en mouvement alternatif vertical, de préférence automatiquement, par des moyens moteurs non représentés. L'agitateur traverse le bouchon à l'intérieur d'un manchon 30 par lequel passe un léger courant d'azote sec préservant l'échantillon de toute condensation d'humidité.

La sonde traverse le bouchon avec étanchéité et est reliée à une unité centrale de traitement de données, telle qu'un microprocesseur, qui permet une exploitation automatique des mesures, et commande le déroulement des séquences du procédé.

L'éprouvette 10 est entourée d'une enveloppe réfrigérante 32 également en matière transparente pourvue d'un embout 34 pour l'entrée d'un fluide frigoporteur et d'un embout 36 pour la sortie dudit fluide, respectivement situés à la partie inférieure et à la partie supérieure de l'enveloppe. L'enveloppe 32 est montée en série avec une pompe immergée assurant la circulation du fluide frigoporteur à débit constant.

Un fluide frigoporteur quelconque pourra être utilisé, par exemple de l'éthanol à -80°C. Le fonctionnement de la pompe est commandé par le microprocesseur, comme il sera expliqué par la suite.

L'enveloppe réfrigérante 32 est elle-même entourée d'une chemise étanche 38 sous vide qui assure le calorifugeage de l'éprouvette.

3

La cellule de mesure comprend en outre un dispositif destiné à mesurer l'intensité d'un faisceau lumineux qui a traversé la colonne de carburéacteur contenue dans l'éprouvette 10.

Dans le mode de réalisation représenté à la figure 1, ce dispositif comprend une fibre optique émettrice 40 disposée axialement à l'extérieur de l'éprouvette 10, selon l'axe de cette dernière, de manière que l'une de ses extrémités soit à faible distance du fond 18 de l'éprouvette et que son autre extrémité soit connectée à une source lumineuse, non représentée. Le dispositif comprend également une fibre optique réceptrice 42 montée axialement à l'intérieur de l'éprouvette, de manière que son extrémité inférieure soit légèrement immergée sous la surface du carburéacteur 23, de préférence à la distance focale de la lentille constituée par le double fond 16, 18 de l'éprouvette et du carburéacteur et que son extrémité extérieure soit reliée à un élément photosensible, non représenté, telle qu'une cellule photo-électrique, destiné à transformer l'intensité lumineuse captée en signaux électriques qui sont traités par le microprocesseur, comme on l'expliquera par la suite.

Avantageusement, l'élément photosensible ne subit pas l'influence de la lumière ambiante en utilisant une source de lumière modulée. On a ainsi la possibilité de travailler en lumière ambiante.

L'appareil nécessite également une source de fluide gazeux, par exemple d'azote sec, reliée à une buse 44 qui débouche sous le fond 18 de l'éprouvette. Un jet d'azote sec est soufflé en continu de manière à éviter la formation sur le fond 18 de givre pouvant conduire à des mesures optiques erronées.

Le réchauffement du carburéacteur, après que la phase de refroidissement ait pris fin, est effectué par mise en circulation dans la cellule d'un gaz, par exemple de l'azote, fourni par une source non représentée. L'admission de l'azote est également commandée par le microprocesseur.

En pratique, l'appareil selon l'invention peut se présenter sous la forme d'une armoire comprenant un logement réservé à la cellule de mesure. Sur la face avant de l'armoire peuvent être montés un interrupteur marche/arrêt, un synoptique indiquant l'état d'avancement de la détermination, un module "Agitation", un bouton "Départ", un bouton "Remise à zéro", un module "Alarmes", une imprimante, un affichage numérique de la température instantanée, et le microprocesseur. L'appareil nécessite à l'extérieur une réserve de fluide frigoporteur, une pompe de mise en circulation de ce fluide, et une réserve d'azote.

L'appareil selon l'invention fonctionne de la façon suivante : Un opérateur met en marche l'appareil en amenant l'interrupteur sur la position marche. La pompe de mise en circulation du fluide frigoporteur se met alors en marche et fournit à la cellule un débit de fluide constant. Le carburéacteur se refroidit alors progressivement à une valeur typique de $1°C/minute$ et sa température ainsi que l'état de transmission de la lumière sont mesurés à des intervalles de temps réguliers. Une horloge est prévue pour permettre à la sonde 18 et au détecteur à fibres optiques 40, 42, d'effectuer respectivement les mesures de la température et de contrôle de l'état de transmission de la lumière, par exemple une fois par seconde. Ces mesures sont transformées en signaux qui sont envoyés au microprocesseur en vue de leur exploitation.

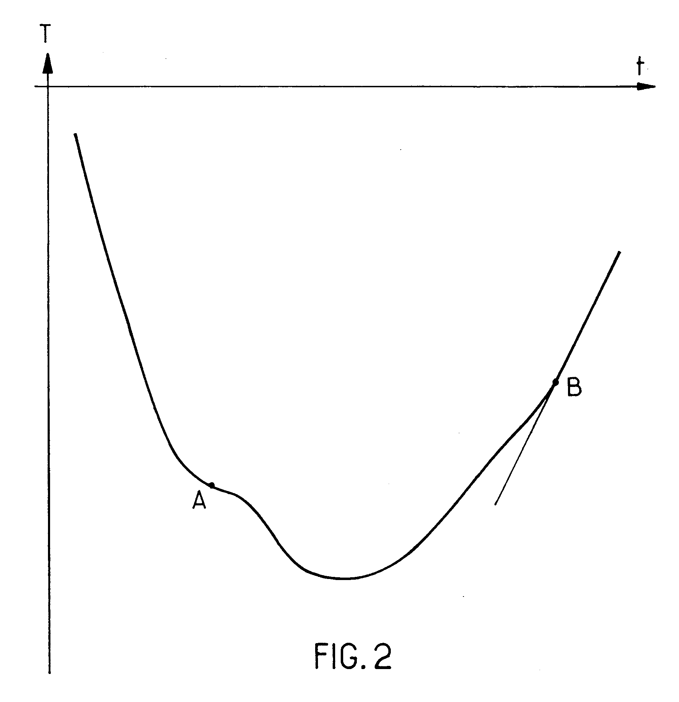

Lorsque des cristaux apparaissent dans le carburéacteur, ils modifient par diffraction l'état de transmission de la lumière. Simultanément, sur la courbe de température/temps représentée à la figure 2, un palier A apparaît sur la branche descendante. Le microprocesseur enregistre alors la température d'apparition de cristaux par détection optique, que l'on notera AO et il calculera au moyen d'un algorithme, que l'on donnera plus loin, la température d'apparition des cristaux, par détection thermique, soit AT.

Une fois ces températures mesurées, le microprocesseur commande l'arrêt de la circulation du fluide frigoporteur et la mise en circulation de l'azote. Le carburéacteur se réchauffe alors progressivement ; la température et l'état de transmission de la lumière sont mesurés toutes les secondes jusqu'à ce que les cristaux aient disparu. Ce moment est atteint lorsque la transmission de la lumière reprend son état initial. A ce moment, l'on atteint sur la branche ascendante de la courbe de la figure 2, le point B où la pente atteint une valeur maximale avant de devenir constante. Le microprocesseur acquiert alors la température de disparition des cristaux par détection optique, soit DO et calcule ladite température, par détection thermique, soit DT. Il procède ensuite aux vérifications suivantes:

$|AT - AO| < r$, r étant la répétabilité de la méthode manuelle normalisée, que l'on prend en général égale à $0,7°C$.

Si cette condition n'est pas satisfaite une pollution du carburéacteur est suspectée, et un message correspondant est imprimé et affiché.

Une vérification analogue est effectuée en ce qui concerne la quantité $|DT - DO|$. Si cette quantité est supérieure à r une pollution du carburéacteur est suspectée.

Enfin, ces deux conditions étant satisfaites, le microprocesseur vérifie encore que :

4

$$\frac{DT + DO}{2} - \frac{AT + AO}{2} < 3°C$$

Si cette condition est réalisée, le message "Point de décongélation

$$\frac{DT + DO}{2}"$$

est imprimé et sinon un message correspondant est affiché, ce qui invite à remettre à zéro l'appareil et à recommencer l'analyse du carburéacteur.

Dans le cas où aucun phénomène n'est observé après que la température ait été abaissée jusqu'à -65°C, un message "Point de décongélation < 60°C" est imprimé.

Dans le cas très particulier où l'appareil mettrait en évidence une pseudo-apparition de cristaux selon la détection thermique, mais sans détection optique, un problème de vitesse de refroidissement est à suspecter (arrêt de l'alimentation en fluide frigoporteur). Un message signalant cette anomalie est alors imprimé.

On expliquera à présent l'algorithme utilisé par le microprocesseur pour la détermination des points d'apparition et de disparition des cristaux à partir des données de l'analyse thermique.

L'enregistrement du temps t et de la température T (au centième de degré Celsius près) est effectué toutes les secondes. Avec ces enregistrements, on peut tracer la courbe $T = f(t)$ représentée à la figure 2. Deux enregistrements successifs étant pratiquement confondus à l'échelle choisie pour le tracé de cette courbe, on considèrera, dans le raisonnement qui va suivre, des enregistrements pris à des intervalles de temps plus longs, par exemple toutes les 20 secondes.

Soit donc t-20, t et t + 20 trois instants successifs séparés l'un de l'autre de 20 secondes, et $T_{t-20}$, T et $T_{t+20}$ les températures correspondantes.

Etant donné que les points correspondants sur la courbe 2 sont relativement rapprochés, on peut admettre en première approximation que les quantités

$$\frac{T_{t+20} - T_t}{20}$$

et

$$\frac{T_t - T_{t-20}}{20}$$

sont respectivement égales à la dérivée première $\frac{dT}{dt}$ de la fonction $T = f(t)$ aux instants t et t-20.

De même, la quantité

$$\frac{(T_{t+20} - T_t) - (T_t - T_{t-20})}{20 \times 20}$$

est sensiblement égale à la dérivée seconde

$$\frac{d^2T}{dt^2}$$

de ladite fonction à l'instant t - 20.

Le début du palier A, créé par l'apparition des cristaux, correspond à une variation notable de la vitesse de refroidissement du produit, ce qui se traduit par un extremum de la courbe dérivée seconde. Quant au point de disparition des cristaux B, il correspond à une vitesse de réchauffement maximum avant que celle-ci ne devienne quasiment constante, ce qui se traduit par un autre extremum sur la courbe dérivée seconde.

Ainsi, pour déterminer les points A et B, le microprocesseur pourrait rechercher les points extrema de la dérivée seconde. Mais une telle détermination est très imprécise parce que la fonction

$$\frac{d^2 T}{dt^2}$$

varie très peu autour de zéro.

Pour éliminer cette difficulté, on fera les remarques suivantes:

1.- Le microprocesseur déterminera les points recherchés non plus à partir de la dérivée seconde, mais à partir de la fonction $Z = (T_{t+20} - T_t) - (T_t - T_{t-20})$ qui lui est proportionnelle. A chaque instant, la valeur prise par cette fonction est en effet 400 fois plus grande que la valeur de la dérivée seconde calculée au même instant. Les variations de la fonction Z étant plus accentuées que celles de la dérivée seconde, il est clair que l'on pourra déterminer plus facilement les point recherchés à partir de cette fonction.

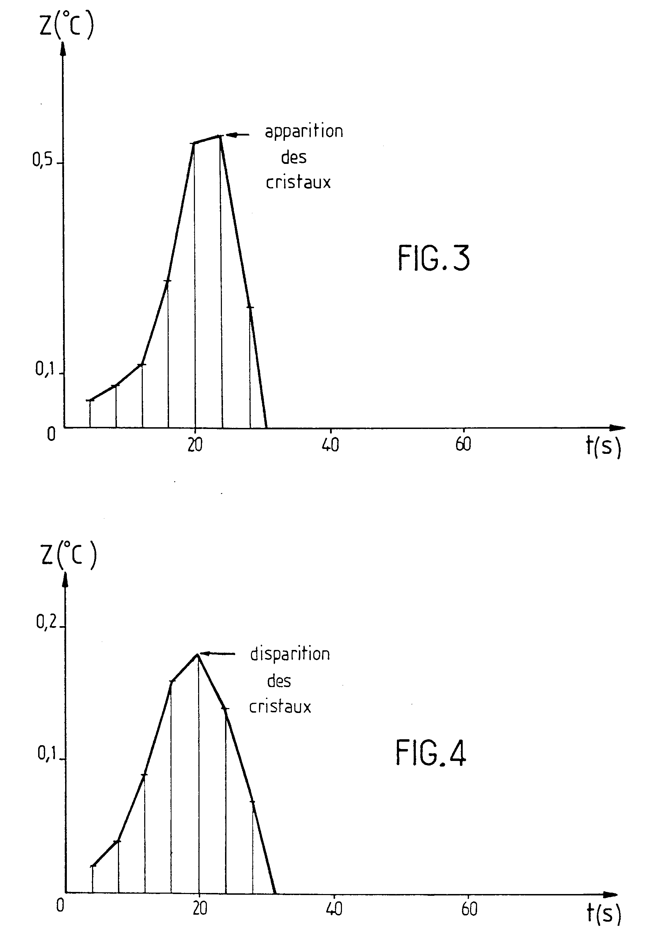

2.- Les figures 3 et 4 montrent les valeurs de |Z| calculées toutes les secondes par le microprocesseur et reportées toutes les quatres secondes, respectivement au voisinage du point d'apparition et du point de disparition des cristaux. Pour la disparition des cristaux, la recherche de ce maximum devra être effectuée sur un intervalle glissant de 4°C.

Dès que le microprocesseur a découvert les extrêm de |Z| il procède aux vérifications signalées précédemment sur les valeurs correspondantes de la température par la détection thermique et par la détection optique et s'il trouve que ces valeurs remplissent bien les conditions définies ci-dessus (différence entre ces températures inférieure à 3°C et répétabilité inférieure à 0,7°C) il commande leur impression.

On donnera à présent un exemple de calcul de la répétabilité estimée, pour un échantillon typique de carbureacteur.

Par le procédé manuel classique on trouve un point de décongélation de -53,5°C.

Dix mesures effectuées par le procédé selon l'invention ont donné les valeurs suivantes pour le point de décongélation T.

| | |
|---|---|
| - 53,03°C | - 52,90°C |
| - 52,93°C | - 52,87°C |
| - 52,98°C | - 52,82°C |
| - 52,93°C | - 52,98°C |
| - 52,98°C | - 52,84°C |

La valeur moyenne de ces mesures est

$$T^* = \frac{\sum T_i}{n} = -52,96°C$$

et l'écart type est

$$s = \sqrt{\frac{\sum (T_i - T^*)^2}{n - 1}} = 0,068.$$

On sait que la répétabilité estimée $\underline{r}$ est donnée par la formule :

$r = t \sqrt{2}\, s$

où t est le facteur de Student. Si l'on souhaite 95% de niveau de confiance les tables donnent t = 2,262.

Il en résulte que r = 0,2°C.

La répétabilité par le procédé selon l'invention est donc bien inférieure à celle du procédé manuel (0,7°C). On pourra adopter comme point de décongélation la valeur arrondie - 53°C.

En conclusion, le procédé selon l'invention permet de définir le point de décongélation avec une plus grande précision qu'avec le procédé manuel. En effet, le temps d'occupation d'un opérateur est de dix minutes au maximum pour la détermination par le procédé selon l'invention, alors qu'il faut bien une heure environ en manuel, correspondant à une présence constante de l'opérateur pendant toute la mesure.

D'autre part, le procédé à double détection permet d'éliminer les erreurs inhérentes aux procédés à simple détection, thermique ou optique.

**Revendications**

1. Procédé pour la détermination automatique du point de décongélation d'un carburéacteur, qui se caractérise en ce qu'il consiste :
   - à soumettre un échantillon du carburéacteur à un cycle thermique au cours duquel la température est diminuée jusqu'à la température d'apparition des cristaux, puis est relevée de façon que les cristaux disparaissent,
   - à effectuer une analyse thermique dudit carburéacteur en mesurant à des intervalles de temps réguliers, au cours dudit cycle, la température que prend le carburéacteur,
   - à effectuer simultanément une analyse optique du carburéacteur en mesurant aux mêmes moments l'intensité de lumière transmise à travers ledit échantillon,
   - à transformer lesdites mesures respectivement en signaux de détection thermique et en signaux de détection optique, et
   - à envoyer lesdits signaux à une unité centrale de traitement des données, telle qu'un microprocesseur, qui calcule à partir de ces signaux les températures d'apparition de cristaux, par détection thermique et par détection optique, et les températures de disparition de cristaux par ces deux types de détection, pour déduire à partir de ces valeurs le point de décongélation dudit carburéacteur.

2. Procédé selon la revendication 1, caractérisé en ce qu'en l'absence de l'apparition de cristaux, le refroidissement de la cellule est poursuivi jusqu'à ce que la température de l'échantillon ait été abaissée jusqu'à -65°C.

3. Procédé selon la revendication 1, caractérisé en ce que l'unité de traitement vérifie que les températures d'apparition de cristaux déterminées par les deux types de détection ne diffèrent pas entre elles de plus d'une valeur prédéterminée, égale à la répétabilité du procédé, et en ce qu'une vérification analogue est effectuée pour les températures de disparition de cristaux déterminées par les deux types de procédés.

4. Procédé selon la revendication 1, caractérisé en ce que l'unité de traitement vérifie si la moyenne entre les températures de disparition de cristaux par la détection thermique et par la détection optique, et la moyenne entre les températures d'apparition de cristaux par ces deux types de détection ne diffèrent pas de plus d'une seconde valeur prédéterminée, fixée par les normes.

5. Procédé selon l'une des revendications précédentes, caractérisé en ce que l'unité centrale de traitement recherche les températures d'apparition et de disparition des cristaux, par détection thermique, selon l'algorithme suivant:
   - l'unité centrale enregistre, toutes les secondes la température $T_t$ du carburéacteur au cours de son refroidissement,
   - elle calcule en permanence la quantité $Z = (T_{t-\Delta t} - T_t) - (T_t - T_{t+\Delta t})$ représentative des variations de la dérivée seconde de la température en fonction du temps, $T_{t-\Delta t}$, $T_t$ et $T_{t+\Delta t}$ étant respectivement les températures relevées à des instants $t - \Delta t$, $t$, et $t + \Delta t$ espacés entre eux d'un intervalle de temps $\Delta t$ relativement faible,
   - elle recherche les températures pour lesquelles $Z$ atteint ses extrema respectivement dans la phase de refroidissement et dans la phase de réchauffement, ces températures étant respectivement les températures d'apparition et de disparition des cristaux par détection thermique.

6. Appareil pour la détermination du point de décongélation d'un carburéacteur par le procédé selon l'une

EP 0 192 551 B1

des revendications précédentes, caractérisé en ce qu'il comprend:
- une éprouvette (10) en matière transparente contenant une quantité prédéterminée du carburéacteur (23), dans laquelle plongent un agitateur (26) et une sonde de température (28) ;
- une source de fluide caloporteur destiné à réchauffer l'éprouvette, dès que la température d'apparition de cristaux a été détectée ;
- une enveloppe réfrigérante (32) également en matière transparente, qui entoure ladite éprouvette et dans laquelle est entretenue au moyen d'une pompe, une circulation de fluide frigoporteur ;
- un détecteur optique comprenant une source émettrice d'un faisceau de lumière conduit par une première fibre optique (40) et un dispositif récepteur de l'intensité lumineuse transmise, conduite par une seconde fibre optique (42), ces deux fibres optiques étant disposées dans l'axe de l'éprouvette (10), la première à l'extérieur de l'éprouvette du côté du fond (18) et la seconde à l'intérieur de celle-ci juste sous la surface du carburéacteur (23), et
- une unité centrale de traitement des données, telle qu'un microprocesseur, destinée à calculer d'une part, les températures d'apparition de cristaux à partir des mesures fournies par la sonde de température et des mesures fournies par le détecteur optique, et d'autre part, les températures de disparition des cristaux à partir de ces deux types de mesures et d'en déduire le point de décongélation dudit carburéacteur.

7. Appareil selon la revendication 6, caractérisé en ce que les fibres optiques (40 et 42) sont reliées respectivement à des éléments photosensibles, la fibre optique émettrice (40) étant montée axialement à l'extérieur de l'éprouvette, du côté du fond de celle-ci, et la fibre optique réceptrice (42) étant disposée axialement à l'intérieur de l'éprouvette de manière que son extrémité réceptrice soit immergée sous la surface du carburéacteur et se trouve au foyer de la lentille constituée par le fond de l'éprouvette et le carburéacteur.

8. Appareil selon l'une des revendications 6 et 7, caractérisé en ce qu'il comprend une horloge permettant d'effectuer des mesures de température et un contrôle de l'état de transmission de la lumière à des intervalles de temps réguliers à l'aide respectivement de la sonde de température (28) et du détecteur optique.

9. Appareil selon l'une des revendications 6 à 8, caractérisé en ce qu'il se présente sous forme d'armoire dans laquelle sont intégrées la cellule de mesure, l'unité centrale de traitement, et en ce que sur la face avant de ladite armoire sont prévus, un interrupteur marche/arrêt, un synoptique indiquant l'état d'avancement de la détermination, un module agitation, un module alarme, un bouton départ, un bouton de remise à zéro du processus, une imprimante et un affichage numérique de la température instantanée, alors qu'une source de fluide frigoporteur, une pompe de mise en circulation de ce fluide et une source de fluide caloporteur sont disposées à l'extérieur de ladite armoire.

**Claims**

1. A method of automatically determining the thaw point of a jet fuel, which is characterised in that it comprises:
- subjecting a sample of the jet fuel to a thermal cycle during which the temperature is decreased to the crystal-formation temperature and is then raised so that the crystals disappear,
- carrying out a thermal analysis of said jet fuel by measuring at regular intervals of time, during said cycle, the temperature of the jet fuel,
- carrying out simultaneously an optical analysis of the jet fuel by measuring at the same instants the intensity of the light transmitted through said sample,
- converting said measurements respectively into thermal detection signals and optical detection signals, and
- feeding said signals to a central data-processing unit, such as a microprocessor, which calculates from these signals the crystal-formation temperatures, by thermal detection and by optical detection, and the crystal-disappearance temperatures by these two types of detection, so as to deduce from these values the thaw point of said jet fuel.

2. A method according to claim 1, characterised in that, in event of the non-appearance of crystals, the cooling of the cell is continued until the temperature of the sample is lowered to -65°C.

8

**3.** A method according to claim 1, characterised in that the processing unit verifies that the crystal-formation temperatures determined by the two types of detection do not differ from one another by more than a predetermined value equal to the repeatability of the method, and in that an analog verification is carried out for the crystal-disappearance temperatures determined by the two types of method.

**4.** A method according to claim 1, characterised in that the processing unit verifies whether the average between the crystal-disappearance temperatures by thermal detection and by optical detection, and the average between the crystal-formation temperatures by these two types of detection do not differ by more than a second predetermined value set by standards.

**5.** A method according to any one of the preceding claims, characterised in that the central processing unit searches for the crystal formation and crystal-disappearance temperatures, by thermal detection, in accordance with the following algorithm:
- the central unit records every second the temperature $T_t$ of the jet fuel while it is being cooled,
- it continuously calculates the quantity $Z = (T_{t-\Delta t} - T_t) - (T_t - T_{t+\Delta t})$ which is representative of the variations in the second derivative of the temperature as a function of time, $T_{t-\Delta t}$, $T_t$ and $T_{t+\Delta t}$ being respectively the temperatures recorded at instants $t - \Delta t$, $t$, and $t + \Delta t$ spaced apart by a relatively short interval of time $\Delta t$,
- it searches for the temperatures for which $Z$ reaches its limits respectively in the cooling phase and in the reheating phase, said temperatures being respectively the crystal-formation temperatures and crystal-disappearance temperatures by thermal detection.

**6.** An apparatus for determining the thaw point of a jet fuel by the method according to any one of the preceding claims, characterised in that it comprises:
- a test tube (10) of transparent material containing a predetermined amount of jet fuel (23), in which an agitator (26) and a temperature sensor (28) are immersed;
- a supply of heat-transfer fluid intended to reheat the test tube as soon as soon as the crystal-formation temperature has been detected;
- a cooling jacket (32), also of transparent material, which surrounds said test tube and in which a circulation of cold-transfer fluid is maintained by means of a pump;
- an optical detector comprising a source transmitting a beam of light conveyed through a first optical fibre (40) and a device for receiving the transmitted luminous intensity, conveyed through a second optical fibre (42), these two optical fibres being disposed on the axis of the test tube (10), the first one outside the test tube at the bottom end (18) and the second one inside the test tube just below the surface of the jet fuel (23), and
- a central data-processing unit, such as a microprocessor, intended to calculate firstly the crystal-formation temperatures from the measurements provided by the temperature sensor and the measurements provided by the optical detector, and secondly the crystal-disappearance temperatures from these two types of measurements, and to deduce therefrom the thaw point of said jet fuel.

**7.** Apparatus according to claim 6, characterised in that the optical fibres (40 and 42) are connected respectively to photosensitive elements, the transmitting optical fibre (40) being mounted axially outside the test tube at the bottom end thereof, and the receiving optical fibre (42) being disposed axially inside the test tube so that its receiving end is submerged below the surface of the jet fuel and is situated at the focus of the lens formed by the bottom of the test tube and the jet fuel.

**8.** Apparatus according to either one of claims 6 and 7, characterised in that it comprises a clock making it possible to carry out temperature measurements and to control the state of light transmission at regular intervals of time, respectively by means of the temperature sensor (28) and the optical detector.

**9.** Apparatus according to any one of claims 6 to 8, characterised in that it is provided in the form of a cabinet, into which are integrated the measuring cell and the central processing unit, and in that on the front surface of said cabinet there are provided an on/off switch, a display indicating the state of advance of the determination, an agitating unit, an alarm unit, a start button, a button for resetting the process to zero, a printer and a numerical display of the instantaneous temperature, whereas a supply of cold-transfer fluid, a pump for circulating said fluid and a supply of heat-transfer fluid are disposed

EP 0 192 551 B1

outside said cabinet.

**Patentansprüche**

1. Verfahren zur automatischen Bestimmung des Taupunktes eines Düsentreibstoffs, dadurch gekennzeichnet:
   - daß eine Probe des Düsentreibstoffs einem Wärmezyklus ausgesetzt wird, in dessen Verlauf die Temperatur bis zum Erscheinen von Kristallen heruntergefahren und dann wieder erhöht wird, bis die Kristalle verschwinden,
   - daß eine thermische Analyse des Düsentreibstoffs durchgeführt wird, indem in regelmäßigen Zeitabständen innerhalb des Zyklus die vom Düsentreibstoff angenommene Temperatur gemessen wird,
   - daß gleichzeitig eine optische Analyse des Düsentreibstoffs erfolgt, indem zu den gleichen Zeiten die Intensität des durch die Probe hindurchgehenden Lichts gemessen wird,
   - daß die Meßwerte jeweils in thermische Erfassungssignale und optische Erfassungssignale umgewandelt werden, und
   - daß die Signale zu einer zentralen Datenverarbeitungsanlage wie zum Beispiel einem Mikroprozessor übertragen werden, wo ausgehend von diesen Signalen die Temperaturen, bei denen Kristalle auftreten, durch thermische Erfassung und durch optische Erfassung und die Temperaturen, bei denen die Kristalle verschwinden, durch diese zwei Arten von Erfassungen errechnet werden, um von diesen Werten den Taupunkt des Düsentreibstoffs abzuleiten.

2. Verfahren nach Anspruch 1, dadurch gekennzeichnet, daß bei fehlender Kristallbildung die Abkühlung der Zelle verfolgt wird, bis die Temperatur der Probe auf -65ºC abgesunken ist.

3. Verfahren nach Anspruch 1, dadurch gekennzeichnet, daß von der Verarbeitungseinheit kontrolliert wird, daß die durch die beiden Arten der Erfassung ermittelten Temperaturen, bei denen sich Kristalle bilden, voneinander um nicht mehr als einen vorbestimmten Wert abweichen, der auf die Wiederholbarkeit des Verfahrens abgestimmt ist, und daß eine anloge Überprüfung der durch die beiden Arten der Erfassung ermittelten Temperaturen erfolgt, bei denen die Kristalle verschwinden.

4. Verfahren nach Anspruch 1, dadurch gekennzeichnet, daß von der Verarbeitungseinheit geprüft wird, ob das Mittel zwischen den Temperaturen, bei denen Kristalle verschwinden, bei der thermischen Erfassung und der optischen Erfassung und das Mittel zwischen den Temperaturen, bei denen Kristalle erscheinen, bei diesen beiden Arten der Erfassung um nicht mehr als um einen zweiten, durch die Normen vorgegebenen Wert voneinander abweichen.

5. Verfahren nach irgendeinem der vorstehenden Ansprüche, dadurch gekennzeichnet, daß die zentrale Verarbeitungseinheit die Temperaturen, bei denen sich Kristalle zeigen und Kristalle verschwinden, durch thermische Erfassung nach folgendem Algorithmus ermittelt:
   - die Zentraleinheit registriert im Abstand von jeweils einer Sekunde die Temperatur $T_t$ des Düsentreibstoffs während dessen Abkühlung,
   - sie errechnet ständig die für die Veränderungen des zweiten Differentialquotienten der Temperatur in Abhängigkeit von der Zeit repräsentative Größe $Z = (T_{t-\Delta t} - T_t) - (T_t - T_{t+\Delta t})$, wobei $T_{t-\Delta t}$, $T_t$ und $T_{t+\Delta t}$ die jeweils zu den Zeitpunkten $t - \Delta t$, $t$ und $t + \Delta t$ erfaßten Temperaturen sind, die durch ein relativ kurzes Zeitintervall $\Delta t$ voneinander abgesetzt sind,
   - sie ermittelt die Temperaturen, bei denen Z die Extremwerte während der Abkühlphase und während der Aufwärmphase erreicht, wobei diese Werte den durch thermische Erfassung ermittelten Temperaturen entsprechen, bei denen Kristalle auftreten und verschwinden.

6. Vorrichtung zur Bestimmung des Taupunktes eines Düsentreibstoffs nach dem in irgendeinem der vorstehenden Ansprüche beschriebenen Verfahren, dadurch gekennzeichnet, daß zu ihr gehören:
   - ein Probengefäß (10) aus durchsichtigem Material mit einer vorbestimmten Menge an Düsentreibstoff (23), in den ein Rührwerk (26) und eine Temperatursonde (28) eingetaucht werden;
   - eine Quelle zur Zuführung eines flüssigen Wärmeübertragungsmittels, um das Probengefäß erneut zu erwärmen, sobald die Temperatur ermittelt worden ist, bei der sich Kristalle zeigen;
   - ein ebenfalls aus durchsichtigem Material hergestellter Kühlmantel (32), der das Probengefäß umgibt und in dem mittels einer Pumpe flüssiges Kühlmittel umgewälzt wird;

10

- ein optischer Detektor mit einer Quelle zur Ausstrahlung eines durch eine erste Lichtleitfaser (40) übertragenen Lichtbündels und einer Vorrichtung zum Empfang der durch eine zweite Lichtleitfaser (42) übertragenen Lichtstärke, wobei diese beiden Lichtleitfasern in der Achse des Probengefäßes (10) liegen und wobei sich die erste Lichtleitfaser an der Außenseite am Boden (18) des Probengefäßes befindet und die zweite Lichtleitfaser im Probengefäß unmittelbar unterhalb der Oberfläche des Düsentreibstoffs (23) angeordnet ist, und

- eine zentrale Datenverarbeitungseinheit, wie zum Beispiel ein Mikroprozessor, um zum einen ausgehend von den durch die Temperaturmeßsonde gelieferten Daten und den vom optischen Detektor stammenden Meßwerten die Temperaturen zu errechnen, bei denen die Kristallbildung einsetzt, und zum anderen ausgehend von diesen beiden Arten von Meßwerten die Temperaturen zu ermitteln, bei denen die Kristalle verschwinden, und hiervon den Taupunkt des Düsentreibstoffs abzuleiten.

7. Vorrichtung nach Anspruch 6, dadurch gekennzeichnet, daß die Lichtleitfasern (40 und 42) jeweils mit lichtempfindlichen Elementen verbunden sind, wobei die emittierende Lichtleitfaser (40) in axialer Richtung außen am Boden des Probengefäßes angeordnet ist und die dem Empfang dienende Lichtleitfaser (42) in axialer Richtung im Innern des Probengefäßes so verläuft, daß ihr Empfängerende unter die Oberfläche des Düsentreibstoffs eingetaucht wird und sich im Brennpunkt der vom Boden des Probengefäßes und vom Düsentreibstoff gebildeten Linse befindet.

8. Vorrichtung nach irgendeinem der Ansprüche 6 und 7, dadurch gekennzeichnet, daß sie einen Zeitgeber umfaßt, mit dem Temperaturmessungen und eine Überprüfung des Zustands der Lichtübertragung mit Hilfe der Temperatursonde (28) und des optischen Detektors in regelmäßigen Zeitabständen möglich sind.

9. Vorrichtung nach irgendeinem der Ansprüche 6 bis 8, dadurch gekennzeichnet, daß sie die Form eines Schranks hat, in den die Meßzelle und die zentrale Verarbeitungseinheit eingebaut sind, und daß sich an der Schrankvorderseite eine EIN/AUS-Schalter, eine Anzeige zur Überwachung des Fortgangs der Temperaturbestimmung, ein Rührmodul, ein Alarmmodul, ein START-Taster, ein Taster zur Rückstellung des Verfahrensablaufs auf Null, ein Drucker und eine numerische Anzeige für die jeweils anstehende Temperatur befinden, während eine Quelle zur Zuführung von flüssigem Kühlmittel, eine Pumpe zur Umwälzung dieses Mediums und eine Quelle zur Zuführung von flüssigem Wärmeübertragungsmittel außerhalb des Schranks angeordnet sind.

FIG.1

FIG. 2

FIG.3

FIG.4